# EUROPÄISCHE PATENTSCHRIFT

(11) **EP 3 905 017 B1**
(45) Veröffentlichungstag und Bekanntmachung des Hinweises auf die Patenterteilung: **28.01.2026**
(21) Anmeldenummer: 21020270.1
(22) Anmeldetag: 29.08.2014
(51) Int. Cl.: G06F 3/0481, G06F 3/0484, G06F 3/0488, G16H 40/63, A61M 16/00

(54) **VERFAHREN UND VORRICHTUNG ZUR BEDIENUNG VON BEATMUNGSGERÄTEN**
METHOD AND DEVICE FOR OPERATING VENTILATORS
PROCÉDÉ ET DISPOSITIF DE FONCTIONNEMENT DES APPAREILS RESPIRATOIRES

(30) Priorität: 29.08.2013 DE 102013014303; 28.03.2014 DE 102014004448
(43) Veröffentlichungstag der Anmeldung: 03.11.2021
(62) Teilanmeldung aus: 14781806.6
(73) Patentinhaber: Löwenstein Medical Technology S.A., 2557 Luxembourg (LU)
(72) Erfinder: FELDHAHN, Karl-Andreas, 22761 Hamburg (DE); SCHRÖTER, Christof, 76307 Karlsbad (DE); RENSMANN, Andreas, 76137 Karlsruhe (DE); STREMPEL, Uwe, 75177 Pforzheim (DE); SCHÄFER, Regina, 76187 Iffezheim (DE); SCHWAIBOLD, Matthias, 76228 Karlsruhe (DE)
(74) Vertreter: Löwenstein Medical IP

(56) Entgegenhaltungen:
- EP-A2- 2 564 887
- US-A- 6 158 432
- PARKS: "HTML 5 RANGE tag", 10 February 2014 (2014-02-10), XP055758421, Retrieved from the Internet <URL:https://web.archive.org/web/20140322022659/https://www.bauer.uh.edu/parks/slider.htm> [retrieved on 20201209]

## Beschreibung

Die Erfindung betrifft eine Bedienvorrichtung für ein Beatmungsgerät.

Beatmungsgeräte weisen üblicherweise getrennte Bedien- und Informations- oder Anzeigeelemente auf. Bedienelemente werden beispielsweise als Schalter oder Drehknöpfe ausgeführt. Die über die Bedienelemente erfolgten Einstellungen können dann an separaten Anzeigen abgelesen werden. Daraus resultiert eine komplexe Bedienung für den Anwender mit wenig intuitiven Menüführungen.

US 6 158 432 A beschreibt ein Beatmungsgerät mit einem berührungsempfindlichen Bildschirm, auf dem Zahlenwerte numerisch und grafisch angezeigt werden können.

EP 2 564 887 A2 beschreibt ein Beatmungsgerät mit einem berührungsempfindlichen Bildschirm, auf dem Kurven von Beatmungsparametern in ihrer Form durch Berühren und Ziehen mit einem Finger angepasst werden können.

Im Internetartikel "HTML 5 RANGE tag" wird beispielhaft beschrieben, wie Schieberegler zum Einstellen von Zahlenwerten in HTML codiert werden können (Parks: "HTML 5 RANGE tag", 10. Februar 2014, XP055758421; URL: https://web.archive.org/web/20140322022659/https://www.bauer.uh.edu/parks/slider. htm [gefunden am 9. Dezember 2020]).

Aufgabe der vorliegenden Erfindung ist es daher, eine anwenderfreundliche und intuitiv bedienbare Bedienvorrichtung für ein Beatmungsgerät bereitzustellen. Insbesondere soll der Anwender wichtige Einstellungen schnell vornehmen können, ohne tief durch verschachtelte Menüstrukturen navigieren zu müssen.

Die Aufgabe wird durch die Merkmale des Hauptanspruchs gelöst.

Die Erfindung betrifft eine Bedienvorrichtung für ein Beatmungsgerät. Die Bedienvorrichtung umfasst: eine berührungsempfindliche grafische Anzeige, die zumindest zeitweise den Wertebereich für einen Beatmungsparameter repräsentiert und zumindest einzelne Werte numerisch darstellt; einen Speicher für Beatmungsparameterwerte; mindestens einen mit dem Wertebereich verbundenen Datenpunkt; mindestens eine mit dem Datenpunkt schaltlogisch verbundene Stelle der berührungsempfindlichen grafischen Anzeige; eine Schaltlogik, die bei Berührung der dem Datenpunkt schaltlogisch zugeordneten Stelle der berührungsempfindlichen grafischen Anzeige eine Anzeige von mindestens einem dem Datenpunkt zugeordneten Zahlenwert und /oder eines Bestätigungsfeldes für den Zahlenwert veranlasst; eine Schaltlogik, die diesen Zahlenwert bei Berührung des Zahlenwertes oder des Betätigungsfeldes auf den zugeordneten Atemgasparameter anwendet und mit dem zugeordneten Atemgasparameter in den Speicher schreibt.

Erfindungsgemäß ist neben der berührungsempfindlichen Anzeige lediglich ein weiteres Bedienelement vorgesehen. Dieses Bedienelement ist mechanisch bedienbar und unterscheidet sich somit von den Berührflächen auf dem Display. Erfindungsgemäß wird das Beatmungsgerät über das Bedienelement ein- und ausgeschaltet, sodass eine grundlegende, patientengerechte Therapie mittels lediglich einer Taste aktiviert werden kann. Dazu ist das Bedienelement schaltlogisch mit dem Gebläsemotor verbunden und aktiviert diesen sowie den Speicher, um hinterlegte Therapiedaten, wie Druckwerte, abzurufen und anzuwenden. Ergänzend kann über die Eingabetasten der berührungsempfindlichen Anzeige eine Anpassung einzelner Werte, wie Druckwerte, erfolgen. Eine Bedienung über die Eingabetasten des Touchscreens ist erfindungsgemäß jedoch nicht notwendig, um die Therapie zu starten.

Die Erfindung betrifft eine Bedienvorrichtung für ein Beatmungsgerät mit einer berührungsempfindlichen grafischen Anzeige und lediglich einem weiteren mechanischen Bedienelement, wobei die grundlegende Therapie durch ein Niederdrücken des mechanischen Bedienelements gestartet werden kann und zusätzliche Einstellungen über eine berührungsempfindliche grafische Anzeige erfolgen.

Erfindungsgemäß ist auch vorgesehen, dass die Fingerposition bereits erkannt wird, wenn man in die Nähe des Bildschirms kommt. Die Bedienlogik wäre ähnlich wie oben beschrieben, jedoch wäre eine Berührung des Bildschirms dann beispielsweise schon die Bestätigung des eingestellten Wertes.

Die Bedienvorrichtung ist auch so ausgebildet, dass der Beatmungsparameter Druck oder Fluss oder Volumen oder Frequenz oder IPAP oder EPAP oder PEEP oder FiO₂ ist.

Die Bedienvorrichtung ist auch so ausgebildet, dass das Bedienfeld in Form eines Zahlenstrahls oder Balkens ausgeführt ist und dort der gesamte Wertebereich in Form eines Zahlenstrahls oder Balkens auf der Anzeige visualisiert ist.

Die Bedienvorrichtung ist auch so ausgebildet, dass der visualisierte Zahlenstrahl auch als Bedienfeld ausgebildet ist.

Die Bedienvorrichtung ist auch so ausgebildet, dass das Bedienfeld über den gesamten visualisierten Einstellbereich berührungsempfindlich ist.

Die Bedienvorrichtung ist auch so ausgebildet, dass der gewünschte Wert mit lediglich einer Berührung des gewünschten Bereiches anwählbar ist.

Die Bedienvorrichtung ist auch so ausgebildet, dass der erkannte Wert in einem Zusatzfeld visualisiert wird.

Die Bedienvorrichtung ist auch so ausgebildet, dass der erkannte Wert zusätzlich mittels der Symbole +/- einer Feineinstellung zugänglich ist.

Die Bedienvorrichtung ist auch so ausgebildet, dass der Speicher zumindest den zuletzt eingegebenen und angewendeten Wert sowie den chronologisch davor eingegebenen und angewendeten Wert speichert und im Falle einer Betätigung der undo-Funktion der Speicher immer den chronologisch jüngsten Wert zuerst ausgibt.

Die Bedienvorrichtung ist auch so ausgebildet, dass zumindest eine in dem Speicher abrufbar gespeicherte Beatmungsparametersammlung zumindest drei voreingestellte Parameter, ausgewählt aus Druck oder Fluss oder Volumen oder Frequenz oder I-PAP oder EPAP oder PEEP oder FiO₂, umfasst und für diese Beatmungsparametersammlung im Bereich der berührungsempfindlichen grafischen Anzeige ein Bereich vorgesehen ist, in dem die Beatmungsparametersammlung grafisch repräsentiert ist, und mindestens einen mit der Beatmungsparametersammlung verbundenen Datenpunkt, mindestens eine mit dem Datenpunkt schaltlogisch verbundene Stelle der berührungsempfindlichen grafischen Anzeige, eine Schaltlogik, die bei Berührung der dem Datenpunkt schaltlogisch zugeordneten Stelle der berührungsempfindlichen grafischen Anzeige eine Anzeige von mindestens einer dem Datenpunkt zugeordneten Beatmungsparametersammlung und/oder eines Bestätigungsfeldes für die Beatmungsparametersammlung veranlasst, eine Schaltlogik, die bei Berührung des Zahlenwertes oder der Beatmungsparametersammlung diese Beatmungsparametersammlung anwendet.

Die Bedienvorrichtung ist auch so ausgebildet, dass die Steuereinheit Strom- oder Widerstands- oder Spannungsänderungen im Bereich des Heizelementes des Anfeuchters registriert und so einen sinkenden oder geringen Wasserstand anhand eines erhöhten Strom- oder Widerstands- oder Spannungswertes des Heizelementes erkennt und dies die Steuereinheit veranlasst, ein Symbol oder eine Textmeldung, die einen niedrigen Wasserstand symbolisiert oder benennt, im Bereich der Anzeige einzublenden.

Die Bedienvorrichtung ist auch so ausgebildet, dass das Bediensystem eine grafische Einstellhilfe für mindestens eine Rampensteilheit umfasst.

Die Bedienvorrichtung ist auch so ausgebildet, dass eine grafische Einstellhilfe für die Triggersensitivität vorhanden ist.

Die Bedienvorrichtung ist auch so ausgebildet, dass mindestens ein Einstellelement als ein Drehrad ausgebildet ist.

Die Bedienvorrichtung ist auch so ausgebildet, dass mindestens ein kreisförmiges Anzeigeelement verwendet ist.

Die Bedienvorrichtung ist auch so ausgebildet, dass ein Dreh-Drückknopf verwendet ist.

Die Bedienvorrichtung ist auch so ausgebildet, dass eine Start-Stopp-Bedienfläche verwendet ist.

Die Bedienvorrichtung ist auch so ausgebildet, dass die Start-Stopp-Bedienfläche im Bereich des Touchscreens angeordnet ist.

Die Bedienvorrichtung ist auch so ausgebildet, dass die Start-Stopp-Bedienfläche von einer Gerätesteuerung in unterschiedlichen Gestaltungen generierbar ist.

Die Bedienvorrichtung ist auch so ausgebildet, dass einzelne Werte eines Beatmungsparameters numerisch dargestellt werden und auch der gewählte Wert numerisch dargestellt wird und auch eine grafische Visualisierung des gewählten Beatmungsparameters erfolgt.

Die Bedienvorrichtung ist auch so ausgebildet, dass drei feste Stufen für den Beatmungsparameter vorgesehen sind und diese Stufen mittels der Symbole +/- verstellbar sind, um so den Beatmungsparameter patientenindividuell feiner abzustimmen.

Die Bedienvorrichtung für ein Beatmungsgerät umfasst: eine grafische Anzeige, die zumindest zeitweise den Wertebereich für einen Beatmungsparameter repräsentiert und zumindest einzelne Werte numerisch darstellt; einen Speicher für Beatmungsparameterwerte; mindestens einen mit dem Wertebereich verbundenen Datenpunkt; mindestens eine mit dem Datenpunkt schaltlogisch verbundene Stelle der grafischen Anzeige; eine Schaltlogik, die bei Betätigung der dem Datenpunkt schaltlogisch zugeordneten Stelle des mechanischen Bedienelementes eine Anzeige von mindestens einem dem Datenpunkt zugeordneten Zahlenwert und/oder eines Bestätigungsfeldes für den Zahlenwert veranlasst; eine Schaltlogik, die diesen Zahlenwert bei Betätigung der dem Datenpunkt schaltlogisch zugeordneten Stelle des mechanischen Bedienelementes auf den zugeordneten Atemgasparameter anwendet und mit dem zugeordneten Atemgasparameter in den Speicher schreibt.

Die Bedienvorrichtung ist auch so ausgebildet, dass auf dem grafischen Anzeigeelement die Veränderung der Werte während des Einstellvorganges über das mechanische Bedienelement und/oder der ausgewählte Wert und/oder der zur Verfügung stehende Wertebereich visualisiert wird bzw. werden.

Die Bedienvorrichtung für ein Beatmungsgerät, bevorzugt für ein CPAP-, APAP-, Bilevel- oder Heimtherapie-Beatmungsgerät, ist mit einer Anzeige zur Darstellung von Informationen und zur Darstellung von Bedienfeldern für den Anwender und zumindest einem berührungsempfindlichen Eingabefeld ausgestattet.

**In** den Zeichnungen sind Ausführungsbeispiele der Erfindung schematisch dargestellt. Es zeigen:
- Fig. 1: eine perspektivische Darstellung eines Beatmungsgerätes mit Atemmaske und Atemgasschlauch,
- Fig. 2: eine Anzeige eines Bedien- und Informationssystems,
- Fig. 3: eine Anzeige zur Veranschaulichung eines Untermenüs,
- Fig. 4: ein Bedienfeld in Form eines Zahlenstrahls,
- Fig. 5a, 5b: ein Bedienfeld in Form einer Grafik,
- Fig. 6a, 6b: eine Darstellung ähnlich wie in Fig. 5 zur Veranschaulichung eines weiteren Betriebszustandes,
- Fig. 7a: ein weiteres Bedienfeld in Form einer Grafik,
- Fig. 7b: eine Einstellung einer Druckwellenform,
- Fig. 7c: eine Variante zu Fig. 7b,
- Fig. 8: eine schematische Darstellung einer grafischen Einstellhilfe für eine Rampensteilheit,
- Fig. 9: eine schematische Darstellung einer grafischen Einstellhilfe für die Triggersensitivität,
- Fig. 10: eine grafische Einstellhilfe für mindestens einen Druck,
- Fig. 11: eine ergänzende Darstellung zu den Figuren 7a, 7b,
- Fig. 12: eine Abbildung zur Veranschaulichung eines Verhältnisses von inspiratorischer Zeit zur gesamten Atemzugszeit,
- Fig. 13: eine schematische Darstellung eines kreisförmigen Bedienelementes,
- Fig. 14: eine alternative Ausführungsform und
- Fig. 15: ein mechanisches Bedienelement.

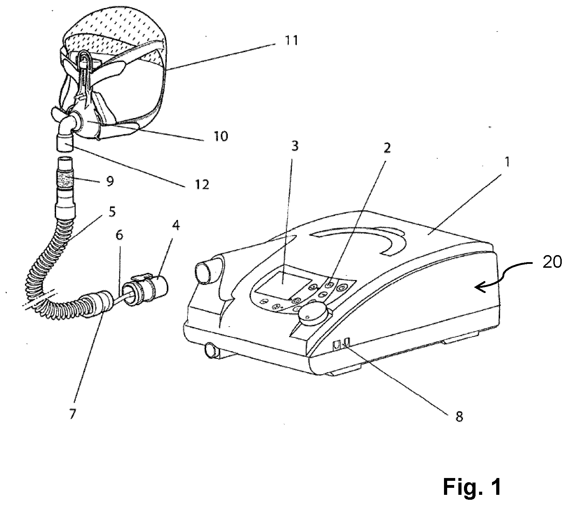

Fig. 1 zeigt den grundsätzlichen Aufbau einer Vorrichtung zur Beatmung. Im Bereich eines Gerätegehäuses 1 eines Beatmungsgerätes 20 mit einer Atemgasquelle im Geräteinnenraum sind ein Bedienelement 2 und ein Bedien- und Informationssystem 3 bestehend aus einer Anzeige 13, einer berührungsempfindlichen Eingabeeinheit 15 mit zumindest einem Bedienfeld 14 angeordnet. Über eine Kopplung 4 ist ein Verbindungsschlauch 5 angeschlossen. Entlang des Verbindungsschlauches 5 kann ein zusätzlicher Druckmessschlauch 6 verlaufen, der über einen Druckeingangsstutzen 7 mit dem Gerätegehäuse 1 verbindbar ist. Zur Ermöglichung einer Datenübertragung weist das Gerätegehäuse 1 zumindest eine Schnittstelle 8 auf. Ein Anfeuchter kann zudem adaptiert werden.

Im Bereich einer dem Gerätegehäuse 1 abgewandten Ausdehnung des Verbindungsschlauches 5 ist ein Ausatmungselement 9 angeordnet. Ebenfalls kann ein Ausatemventil verwendet werden.

Fig. 1 zeigt darüber hinaus ein als Beatmungsmaske 10 ausgebildetes Patienteninterface, das als Nasalmaske realisiert ist. Eine Fixierung im Bereich eines Kopfes eines Patienten kann über eine Kopfhaube 11 erfolgen. Im Bereich seiner dem Verbindungsschlauch 5 zugewandten Ausdehnung weist das Patienteninterface 10 ein Kupplungselement 12 auf.

Über die Schnittstelle 8 kann die Eingabe und/oder Ausgabe von Daten wie beispielsweise Totraumvolumen erfolgen. Die Schnittstellen können kabelgebunden, als Infrarotschnittstelle, Bluetooth-Schnittstelle oder USB realisiert sein. Bevorzugt ist auch ein Kartenschacht vorgesehen. Die Schnittstelle 8 kann auch als LAN-Schnittstelle oder als sonstige Schnittstelle zur Anbindung an das Internet ausgeführt sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern.

Über die Schnittstelle 8, beispielsweise als Kartenschacht oder USB ausgeführt, können auch therapiefremde Daten in das erfindungsgemäße Beatmungsgerät 20 geladen oder von diesem ausgeführt werden. So ist daran gedacht, beispielsweise Fotos oder Videos mittels Speichermedien über die Schnittstelle 8 im Bereich der Anzeige 13 darzustellen. Der Anwender muss - werden vom Gerät 20 externe Speichermedien erkannt - eine Abfrage im Bedienfeld 14 bestätigen, woraufhin die Daten wahlweise im Bereich des Beatmungsgerätes 20 gespeichert oder ausgeführt werden.

Das erfindungsgemäße Beatmungsgerät 20 ist so ausgelegt, dass es über einen Schlauch und ein Patienteninterface mit einem Patienten verbunden werden kann, um eine Beatmung bereitzustellen. Es umfasst eine Quelle für Atemgas, die beispielsweise als ein Elektromotor mit Gebläserad ausgebildet ist, eine Einrichtung zur Ermittlung von Druck und/oder Fluss und/oder Volumen des Atemgases sowie eine Steuereinheit 19, die so ausgelegt ist, dass sie für jeden Atemzyklus auf der Basis eines vorbestimmten Wertes für den Patienten und/oder auf Basis von Messsignalen für die Parameter Druck und/oder Fluss und/oder Volumen einen Atemgasdruck bestimmt und die Quelle für Atemgas derart reguliert, dass der Atemgasdruck erzeugt wird.

Die Steuereinheit 19 ist ferner so ausgelegt, dass sie den aktuellen Druck und/oder Fluss und/oder das Volumen von Atemgas bestimmt und den aktuellen Wert über das mit der Steuereinheit 19 verbundene Bedien- und Informationssystem 3 darstellt. Die Steuereinheit 19 ist außerdem so ausgelegt, dass sie bezogen auf einen oder mehrere Parameter Trendveränderungen ihrer Berechnungen über der Zeit bestimmt, wobei die Trendveränderungen auf der Anzeige 13 angezeigt werden können.

Weiterhin vergleicht die Steuereinheit 19 solche Parameterwerte, die von einem Benutzer vorgegeben wurden, beispielsweise obere und untere Druckgrenzen oder eine maximal tolerierbare Anzahl von Apnoen pro Zeiteinheit oder eine maximal tolerierbare Leckage, mit den aktuellen Werten und generiert eine Benutzerinformation zu Abweichungen von der Vorgabe. Die Benutzerinformation wird bevorzugt über das Bedien- und Informationssystem 3 grafisch visualisiert.

So werden aus dem gemessenen Atemfluss über eine Abnahme des Atem(zeit)volumens für eine Zeitdauer von mindestens 10 s Apnoen und Hypopnoen erkannt. Zusätzlich wird über Druck- und Flussschwankungen Schnarchen sowie über die inspiratorische Flusskontur Flattening erkannt. Daraus werden für jede ausreichend lange nächtliche Therapie Indizes berechnet, nämlich: AHI (= Anzahl der Apnoen und Hypopnoen pro artefaktfreier Therapiedauer), RDI (= Anzahl aller respiratorischen Ereignisse pro artefaktfreier Therapiedauer), Anteil der Atemzüge mit Flattening, Anteil der Atemzüge mit Schnarchen. Bevorzugt werden auch Daten ermittelt, die auf das Nutzungsverhalten oder auf die Nutzungsdauer des Gerätes durch den Patienten schließen lassen. Diese Daten werden täglich oder wöchentlich oder monatlich ermittelt und gespeichert. Bedarfsweise werden die Nutzungsdaten, gegebenenfalls zusammen mit einer Gerätekennung, über eine Internetverbindung oder Mobilfunkverbindung abgerufen und versendet.

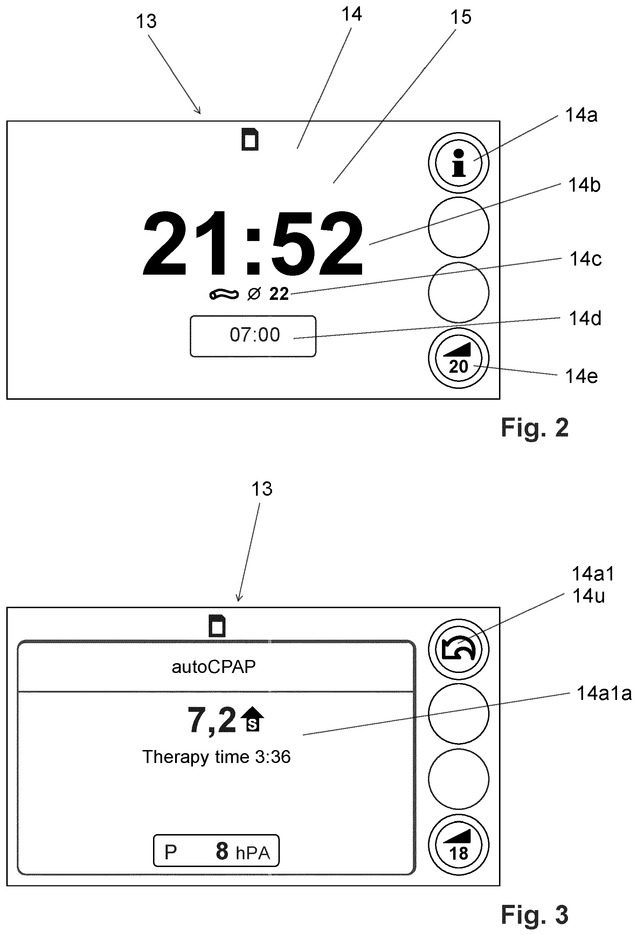

Fig. 2 zeigt das Bedien- und Informationssystem 3 für das Beatmungsgerät 20 mit einer beleuchteten oder hinterleuchteten Anzeige 13 zur Darstellung von Bedienfeldern 14 oder Informationen für den Anwender und der berührungsempfindlichen Eingabeeinheit 15 in räumlicher Nähe zum dargestellten Bedienfeld 14. In einer konkreten Ausführungsform handelt es sich um eine Mensch-Maschine-Schnittstelle in Form eines sogenannten Touchscreens, wobei der Fachmann unterschiedliche Typen kennt, die alle als Bestandteil des Bedien- und Informationssystems 3 infrage kommen.

Im Bereich der Anzeige 13 werden zumindest ein erstes Bedienfeld 14a und ein zweites Bedienfeld 14b dargestellt. Die Steuereinheit 19 ist ausgestaltet, um das Menü auf der Anzeige 13 darzustellen.

Eine Verarbeitungseinheit 18, welche mit der Anzeige 13 und der berührungsempfindlichen Eingabeeinheit 15 gekoppelt ist, ist ausgestaltet, um eine Bedienung des Bedienfeldes 14 über die Eingabeeinheit 15 zu erfassen und in Abhängigkeit davon eine Funktion des Menüs über die Steuereinheit 19 anzusteuern. Bevorzugt wird ein einem Bedienfeld 14b, 14c, 14d ... zugeordnetes Menü auf der Anzeige 13 räumlich benachbart zum Bedienfeld oder an derselben Stelle des Bedienfeldes dargestellt. Eine zeitgleiche oder zeitversetzte Bedienung weiterer Bedienfelder 14b, 14c, 14d ... über die Eingabeeinheit 15 kann ebenfalls über die Verarbeitungseinheit 18 erfasst werden. Die Verarbeitungseinheit 18 veranlasst daraufhin die Steuereinheit 19, das dem gewählten Bedienfeld zugeordnete Menü in der ersten Ebene (= Untermenü) aufzurufen. Bevorzugt wir ein dem Bedienfeld 14b, 14c, 14d ... zugeordnetes Untermenü auf der Anzeige 13 räumlich benachbart zum Bedienfeld oder zum Menü oder an derselben Stelle des Bedienfeldes oder des Menüs dargestellt.

Von der Steuereinheit 19 wird über die Anzeige 13 ein Untermenü dargestellt. Bevorzugt wird das Untermenü im Wesentlichen an derselben Stelle dargestellt wie das Menü, aus dem das Untermenü hervorgeht. Im Untermenü können nun weitere Bedienfelder 14 oder Informationen dargestellt werden. Grundsätzlich ist eine Navigation in mehrere Untermenüs vorgesehen. Bevorzugt ist die Verzweigung jedoch nicht tiefer als zwei Menüebenen. Um aus einem Untermenü wieder in das Menü zurückzukehren, ist immer an derselben Stelle ein Bedienfeld 14 vorgesehen, dessen Betätigung die Steuereinheit 19 veranlasst, auf der Anzeige 13 das Menü der nächsthöheren Hierarchiestufe darzustellen.

In der unteren rechten Ecke ist der aktuell eingestellte Beatmungsdruck in mbar mit einem zugeordneten Bedienfeld 14e dargestellt. In der oberen rechten Ecke ist ein Informationsfeld mit einem zugeordneten Bedienfeld 14a dargestellt.

Wenn beispielsweise der Beatmungsdruck als betätigbare Einstellfunktion geändert werden soll, berührt der Anwender einfach das entsprechende Feld der Anzeige 13, in dem die Information über den aktuellen Druck angezeigt wird, hier das Bedienfeld 14e. Daraufhin wird von der Steuereinheit 19 die Anzeige eines Bedienfeldes veranlasst.

Im einfachsten Fall werden oberhalb und unterhalb oder rechts und links von der gewählten betätigbaren Einstellfunktion zwei Bedienfelder in Form von Plus- und Minussymbolen 14f2, 14f3 visualisiert. Eine Betätigung der Bedienfelder wird von der Verarbeitungseinheit 18 erfasst, welche daraufhin die Steuereinheit 19 veranlasst, den Wert der betätigbaren Einstellfunktionen entsprechend der Eingabe zu verändern und im Bereich der Anzeige 13 zu visualisieren. Der geänderte Parameter wird von der Steuereinheit 19 in dem entsprechenden Feld der Anzeige 13 dargestellt und gleichzeitig oder erst nach Anwenderauswahl über ein Steuersignal an das Gebläse des Beatmungsgerätes 20 als neuer Parameter eingestellt und angewandt. Bevorzugt werden zunächst der Einstellwert und der Ist-Wert visualisiert. Nachdem der Ist-Wert dem Einstellwert entspricht, wird nur noch der Ist-Wert dargestellt.

Die so eingestellten Parameter werden von der Steuereinheit 19 gleichzeitig in einen Speicher geschrieben, welcher als Zwischenspeicher für die aktuell anzuwendenden Parameterwerte dient. Der Speicher speichert immer zumindest die zuletzt eingegebenen und angewendeten Werte. Bei einer Betätigung der undo-Funktion gibt der Speicher immer diese letzten Werte zuerst aus.

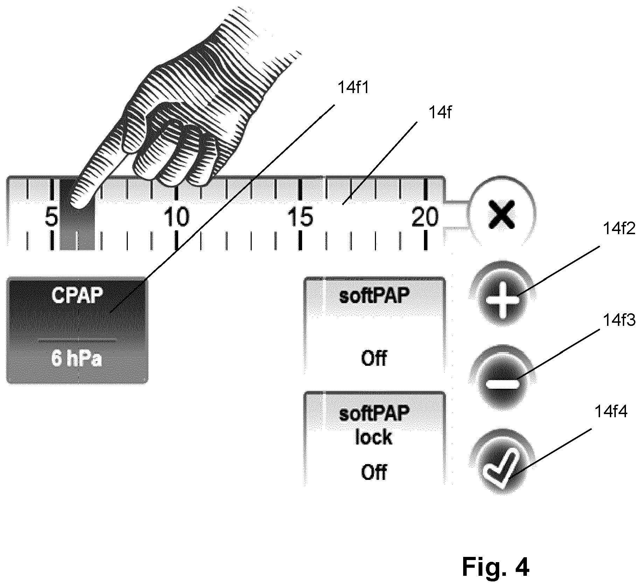

Fig. 4 zeigt ein Bedienfeld 14f in Form eines Zahlenstrahls oder Balkens. Dort kann die Einstellung von Parametern mit vielen Einstellstufen, wie beispielsweise Beatmungsdruck, Frequenz, Fluss, Volumen oder Hintergrundfrequenz (beispielsweise von 6 bis 40 1/min), erfolgen.

Bevorzugt wird für die Einstellung eines Therapiedrucks der gesamte Druckbereich in Form eines Zahlenstrahls oder Balkens auf der Anzeige 13 visualisiert. Zudem ist der visualisierte Zahlenstrahl auch als das Bedienfeld 14f ausgebildet. Das Bedienfeld 14f für den Druck ist über den gesamten visualisierten Einstellbereich berührungsempfindlich. Der Anwender kann daher den gewünschten Wert mit lediglich einer Berührung des gewünschten Druckbereiches anwählen. Der erkannte Wert wird in einem Zusatzfeld 14f1 visualisiert. Sofern der erkannte und visualisierte Wert korrekt ist, kann der Anwender diesen durch eine Berührung des Zusatzfeldes 14f1 zur Anwendung bringen. Zusätzlich ist mittels der Plus- und Minussymbole 14f2, 14f3 eine Feineinstellung mit Schrittweite 0,5 möglich.

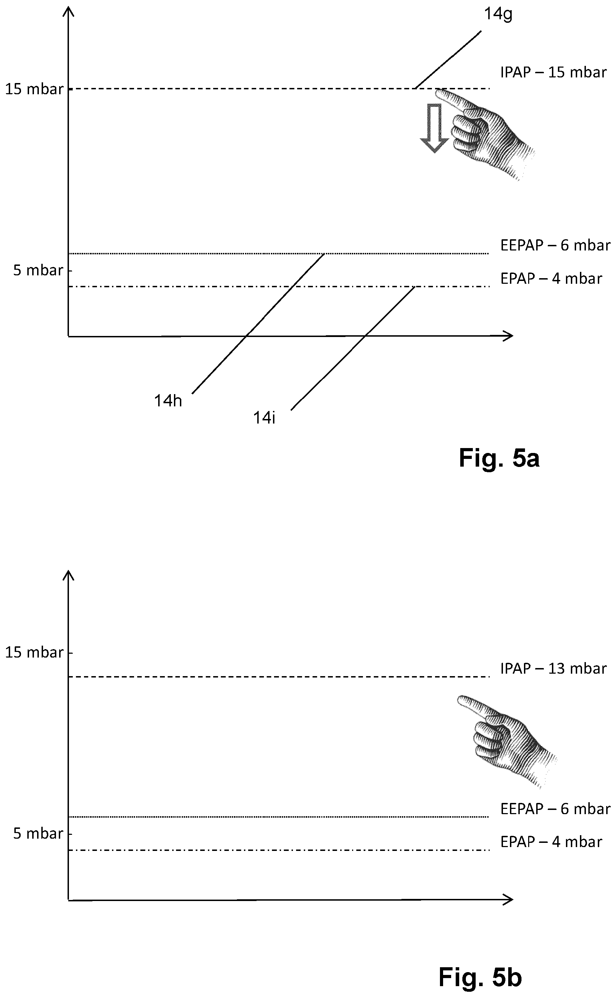

Ein Bedienfeld in Form einer Listenauswahl wird bei der Einstellung vieler Optionen eingeblendet, wie beispielsweise zur Auswahl der Sprache für das Userinterface oder zur Auswahl der Einstellparameter für die Beatmung, wobei die auswählbaren Optionen in Schriftform oder symbolisch angezeigt werden und die Auswahl der gewünschten Option durch Berührung derselben erfolgt, woraufhin die Steuereinheit 19 über ein Steuersignal die Umsetzung der Auswahl veranlasst oder den Einstellbereich darstellt. In Fig. 5a bzw. 5b sind alle Einstellungen für den Nutzer auf einen Blick erkennbar. Jeder Wert kann zur Verstellung direkt durch Berührung ausgewählt werden.

Ein Bedienfeld kann dann in Form eines Zahlenstrahls oder Balkens wie in Fig. 4 visualisiert werden. Dort kann die Einstellung von Parametern durch Berührung des gewünschten Bereiches erfolgen.

Erfindungsgemäß ist vorgesehen, dass edas aktive Bedienfeld 14 durch eine intensivere Farbe hervorgehoben wird, wodurch der Anwender auf die Einstellfunktion hingewiesen wird und nicht aktive Bedienfelder in blasserer Färbung visualisiert werden. Die blassere Färbung weist den Anwender darauf hin, dass die Felder nicht aktiv sind.

Fig. 5a zeigt ein Bedienfeld in Form einer Grafik. Visualisiert werden hier die aktuellen oder gespeicherten Druckwerte für den inspiratorischen Druck IPAP, den exspiratorischen Druck EPAP und das endexspiratorische Druckniveau EEPAP.

Will der Anwender einen dieser Drücke verändern, so muss er eine Linie 14g, 14h, 14i, die den Druckbereich repräsentiert, berühren und den Druckbereich dann auf das gewünschte Niveau bewegen, indem er mit dem Finger über die Anzeige gleitet. Die gewählte Linie wandert dabei mit der Bewegung und zudem wird der Wert mit dargestellt. Beendet der Anwender die Berührung, so wird dieser eingestellte Wert angewendet. Im vorliegenden Beispiel hat der Anwender den IPAP (siehe die Linie 14g in Fig. 5a) von 15 mbar auf 13 mbar reduziert, was in Fig. 5b dargestellt ist.

Ebenso kann der Anwender den EPAP (siehe die Linie 14i in Fig. 5a) und den EEPAP (siehe die Linie 14h in Fig. 5a) einstellen.

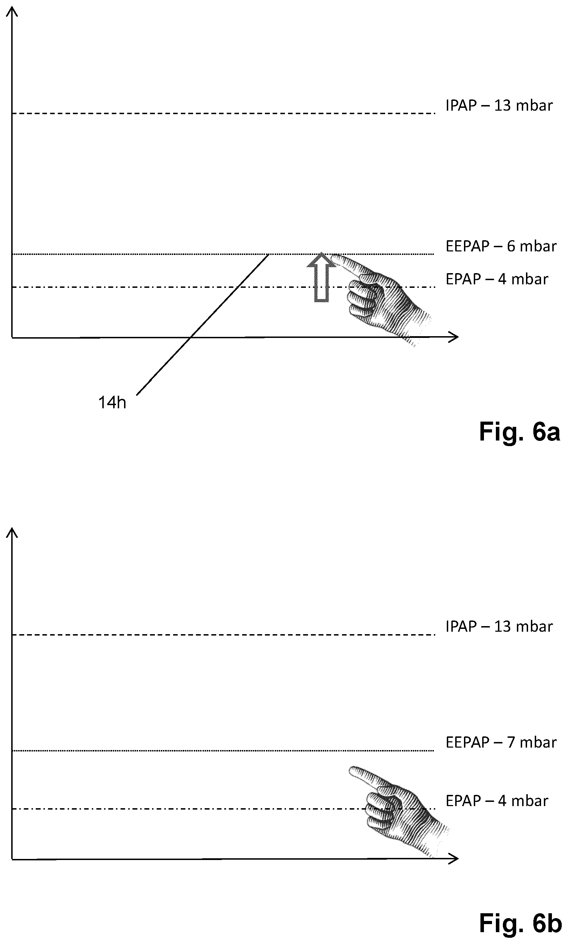

Fig. 6a zeigt ebenfalls ein Bedienfeld in Form einer Grafik. Visualisiert werden hier die aktuellen oder gespeicherten Druckwerte für den inspiratorischen Druck IPAP, den exspiratorischen Druck EPAP und das endexspiratorische Druckniveau EEPAP.

Will der Anwender einen dieser Drücke verändern, so muss er eine Linie 14g, 14h, 14i, die den Druckbereich repräsentiert, berühren und den Druckbereich dann auf das gewünschte Niveau bewegen, indem er mit dem Finger über die Anzeige gleitet. Die gewählte Linie wandert dabei mit der Bewegung und zudem wird der Wert mit dargestellt. Beendet der Anwender die Berührung, so wird dieser eingestellte Wert angewendet. Im vorliegenden Beispiel hat der Anwender den EEPAP (siehe die Linie 14h in Fig. 6a) von 6 mbar auf 7 mbar erhöht, was in Fig. 6b dargestellt ist.

Ebenso kann der Anwender die Steigungen der Druckübergänge und die Druckwellenform einstellen.

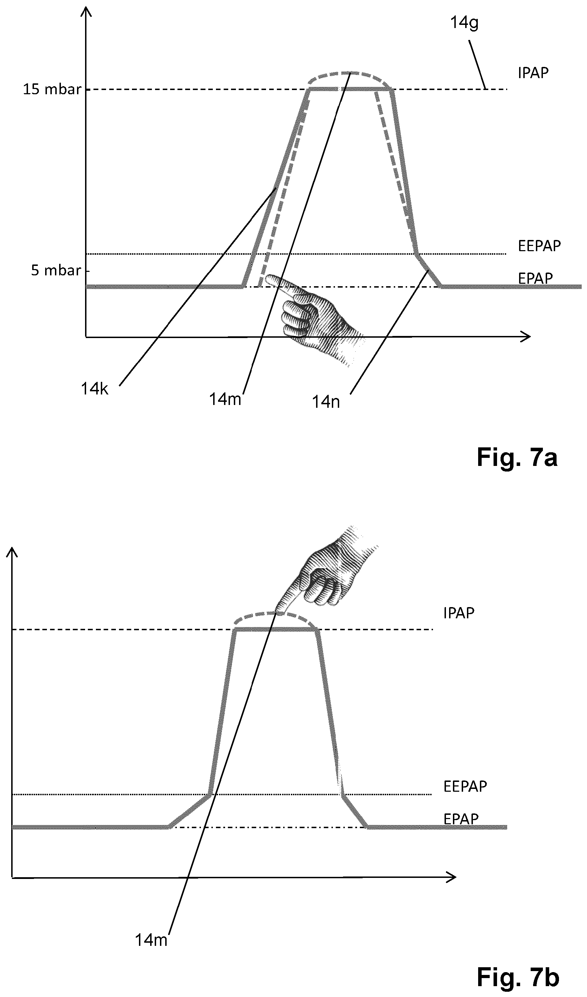

Fig. 7a zeigt ein Bedienfeld in Form einer Grafik. Visualisiert werden hier die aktuellen oder gespeicherten Druckwerte für den inspiratorischen Druck IPAP, den exspiratorischen Druck EPAP und das endexspiratorische Druckniveau EEPAP. Zudem werden die Steigungen der Druckübergänge und die Druckwellenform dargestellt. Die Steigung des Druckübergangs vom EPAP auf den IPAP (siehe die Linie 14k) ist einmal als durchgezogene Linie und einmal als gestrichelte, unterbrochene Linie dargestellt. Die gestrichelte, unterbrochene Linie erscheint, wenn der Anwender die Linie 14k berührt und bewegt.

Will der Anwender die Steigung des Druckübergangs vom EPAP auf den IPAP verändern, so muss er die Linie 14k, die die Steigung repräsentiert, berühren und dann auf die gewünschte Steilheit bringen, indem er die Linie 14k - bevorzugt im Bereich der Anfangs- oder Endpunkte, also nahe beim EPAP oder IPAP -mit dem Finger in horizontaler Richtung verschiebt. Die gewählte Linie 14k wandert dabei als unterbrochene Linie mit der Bewegung mit, wobei der gegenüberliegende Ankerpunkt der Linie fixiert bleibt. Die gewählte Linie erscheint während des Verstellvorgangs beispielsweise gestrichelt oder in einer anderen Farbe. Zudem kann der Wert der Steigung mit dargestellt werden. Beendet der Anwender die Berührung, so wird dieser eingestellte Wert angewendet und die Linie ist wieder durchgezogen.

Fig. 7b zeigt, wie eine Druckwellenform 14m eingestellt wird. Die Druckwellenform 14m ist eine Überhöhung des IPAP (siehe die Linie 14g in Fig. 7a), die der Anwender einstellen kann. Typischerweise ist der IPAP konstant, hier als durchgezogene Linie dargestellt. Auf Anwenderauswahl ist der IPAP jedoch leicht ansteigend auf einen erhöhten IPAP und leicht abfallend auf den IPAP einstellbar, hier als unterbrochene Linie dargestellt.

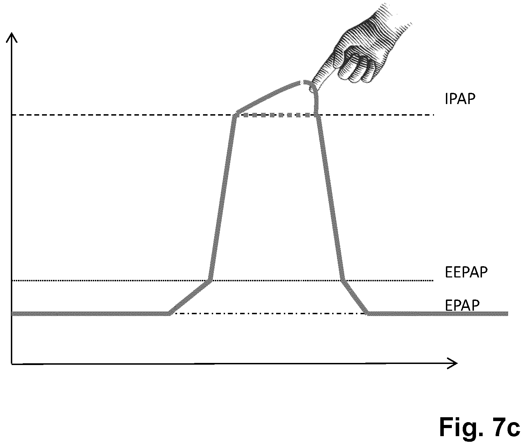

Will der Anwender die Druckwellenform 14m des IPAP verändern, so muss er die Linie 14g, die den IPAP repräsentiert, berühren und dann in vertikaler Richtung bewegen, um so die gewünschte Wellenform einzustellen. In dem Bereich der Berührung wird immer der maximale Druck der Wellenform eingestellt. Die ansteigende und die abfallende Flanke folgen passiv. Wenn der Anwender die Linie 14g im Anfangsbereich (links) berührt und in vertikaler Richtung bewegt, wird die resultierende Wellenform 14m einen steilen Anstieg mit einem Druckmaximum am Anfang haben und dann abfallen. Wenn der Anwender die Linie 14g im Endbereich (rechts) berührt und in vertikaler Richtung bewegt, wird die resultierende Wellenform 14m einen sanften Anstieg mit einem Druckmaximum am Ende haben und dann stark abfallen (siehe Fig. 7c).

Wenn der Anwender die Linie 14g in der Mitte berührt und in vertikaler Richtung bewegt, wird die resultierende Wellenform 14m einen symmetrischen Anstieg und Abfall haben, mit einem Druckmaximum in der Mitte.

Für die Ausführungen zu allen Figuren gilt auch:
Der dargestellte Wertebereich auf dem Zahlenstrahl wird so skaliert, dass er immer genau den Bereich zwischen dem aktuell möglichen Minimum und Maximum für die Einstellung des jeweiligen Parameters umfasst. Dabei werden mögliche Voreinstellungen oder Grenzwerte berücksichtigt.

Alternativ wird immer ein universeller Wertebereich dargestellt und das aktuell gültige Minimum und Maximum (Voreinstellungen oder Grenzwerte) werden gekennzeichnet, beispielsweise durch zusätzliche Linien oder eine Schraffur entweder des gültigen oder des ungültigen Wertebereiches.

Der aktuell bis zum Bestätigen des neuen Wertes noch für die Beatmung aktive alte Wert wird zusätzlich dargestellt. Dies kann beispielsweise durch eine zusätzliche Linie oder eine farbliche Markierung erfolgen.

Der aktuell gewählte Wert kann statt einer farblich hervorgehobenen Linie auch beispielsweise durch einen Pfeil, der von oben oder unten auf die Skala zeigt, oder durch Einfärben einer Zahl auf der Skala markiert werden.

Es können auch zwei oder mehr Werte gleichzeitig in einer Skala eingestellt werden. Beispielsweise die obere und die untere Druckgrenze. Dabei werden beide unterscheidbar dargestellt, beispielsweise durch unterschiedliche Färbung oder Schraffur. Die Umschaltung zwischen der Einstellung des einen und des anderen Wertes erfolgt entweder innerhalb der Skala. Beispielsweise wird immer der der Berührung am nächsten liegende Parameter verstellt. Alternativ muss außerhalb der Skala, beispielsweise durch Aktivierung über die Kacheln, zwischen beiden Parametern hinund hergeschaltet werden. Alternativ ist auch daran gedacht, dass nach Einstellung des ersten Wertes ein sensitives Zeitfenster für die Einstellung des zweiten Wertes verbleibt, sodass ohne Umwege über Menüs auf dem dargestellten Zahlenstrahl beide Werte sukzessiv einstellbar sind.

**In** der Skala müssen nicht immer nur Zahlen stehen. Es könnte auch zwischen verschiedenen Bereichen umgeschaltet werden, die durch Worte oder kombiniert durch Worte und Zahlen beschrieben sind, beispielsweise "Klein - Mittel - Groß - Sehr groß", oder "Aus - Leicht - Normal - Stark", "Aus - 0, 1, 2, ... Max".

Während man Zahlenwerte auf der Zahlenskala einstellt, könnte es separat dazu einen weiteren optischen Feedback-Bereich geben. Beispielsweise wenn die Rampensteilheit für die In-Ex-Rampe eingestellt wird, kann auf dem Display an einer weiteren Position ein Trapez als Vereinfachung eines Druckprofils dargestellt werden, bei dem analog zur Auswahl auf dem Bedienfeld die Rampe steiler oder flacher dargestellt wird.

Es ist auch ein akustisches Feedback vorgesehen. Beispielsweise ist ein Ton oder eine Tonfolge umso lauter, je größer der gerade gewählte Wert auf dem Bedienfeld ist und umgekehrt. Alternativ ist ein farbliches Feedback vorgesehen, beispielsweise eine Farbänderung der Skala oder der Markierung des aktuellen Wertes, wenn dieser bestimmte Werte über- oder unterschreitet.

Folgende Parameter lassen sich noch über ein Bedienfeld darstellen: Rampensteilheiten für den Druckübergang von Inspiration zu Exspiration und umgekehrt, Triggersensitivität, Zeitdauer für die Druckrampe zu Beginn der Therapie (Softstart), Lautstärke akustischer Ausgaben, Helligkeit eines Displays oder von zusätzlichen LEDs oder Anzeigeeinheiten, Empfindlichkeit eines Touchscreens oder bestimmter Algorithmusteile, Target-Volumen, Target-Ventilation, Patientencharakteristika wie Größe, Alter, Gewicht, BMI oder damit in Zusammenhang stehende Werte, Befeuchterstufe, Sollwerte für mindestens eine Temperatur oder Feuchte des Atemgases, Uhrzeit, Datum, Zeitzone, Dauer eines Statistikzeitraums, Größe einer dargestellten Information, Zielwert für eine Compliance in Minuten oder Stunden, mindestens eine subjektive Befindlichkeit eines Patienten.

Statt der linearen Darstellung kann das Bedienfeld auch kreisförmig oder oval sein und damit beispielsweise ein Drehrad simulieren.

Erfindungsgemäß ist auch vorgesehen, dass ein virtueller und drehbarer Steuerknopf oder ein Bildlaufrad angezeigt wird.

Die Zahlenskala muss nicht linear sein. Sie kann auch beispielsweise logarithmisch sein oder im Bereich des aktuellen Wertes höher, ähnlich einer Lupe, und weiter entfernt davon gröber aufgelöst sein.

Alternativ kann auch zwischen einer detaillierteren Darstellung eines Teilbereiches, beispielsweise rings um den aktuellen Wert, und einer Darstellung des Gesamtbereiches umgeschaltet werden.

Um dem jeweiligen Patienten gerecht zu werden, kann die Geschwindigkeit des Druckanstiegs oder Flussanstiegs festgelegt werden. Es wird also festgelegt, wie lange der Druckanstieg vom unteren zum oberen Druckniveau dauert. Dabei wird die Zeit in Sekunden oder beim Fluss der Gasfluss in Liter pro Minute eingestellt. Zu Beginn der Inspiration wird die Atemluft mit einem geringeren als dem eingestellten Fluss verabreicht. Der Gasfluss nimmt im Verlauf der Inspiration bis auf den eingestellten Wert zu. Diese Einstellungen haben unmittelbare Auswirkungen auf das gelieferte Tidalvolumen VT. Die Charakteristik der Druckanhebung oder Druckabsenkung ist einstellbar und erfolgt bevorzugt rampenförmig. Die Druckanhebung kann unter einer gleichmäßigen Rampensteigung auf das erhöhte Druckniveau stattfinden. Die Druckanhebung oder Druckabsenkung kann mit einer veränderlichen Rampensteigung erfolgen. Der Wert des erhöhten oder erniedrigten Druckniveaus ist bevorzugt in mbar-Schritten oder Bruchteilen von mbar einstellbar. Die Rampensteilheit für den Druckanstieg oder Druckabfall kann auf dem Display an einer weiteren Position als eine Grafik dargestellt werden, beispielsweise als ein Trapez als Vereinfachung eines Druckprofils, bei dem analog zur Auswahl auf dem Bedienfeld die Rampe steiler oder flacher dargestellt wird.

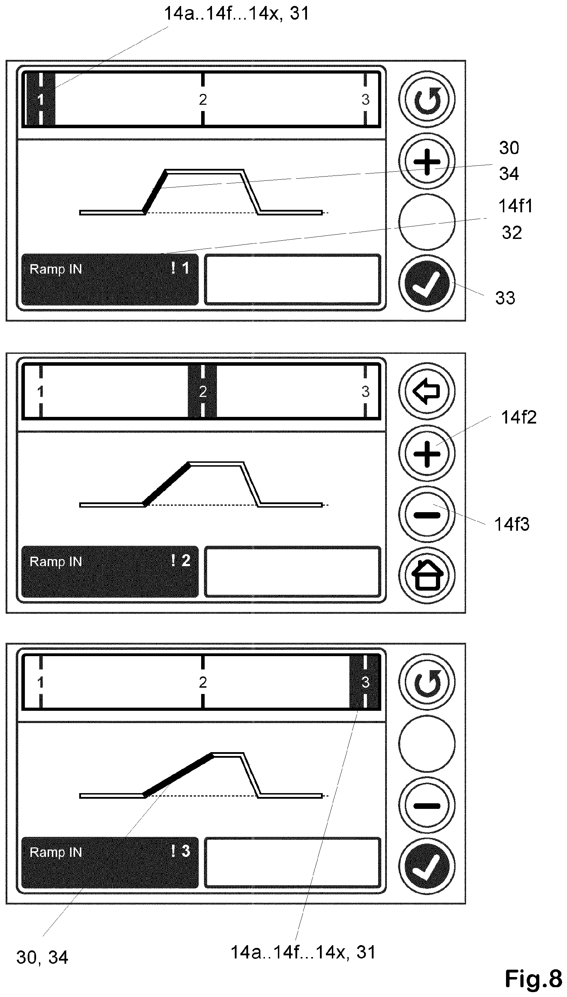

Fig. 8 zeigt eine grafische Einstellhilfe für die Rampensteilheit für den Übergang vom exspiratorischem Druck auf den inspiratorischen Druck (grün markiert). Die aktuelle Stufe kann per Slider oder Lineal oder alternativ mit + und - eingestellt werden. Das Lineal dient zusätzlich mit seiner grünen Markierung nicht nur als Einstellwerkzeug, sondern auch als Anzeigeelement. Dies bietet den Vorteil, dass der Wert auch ungeübten Nutzer direkt bei der Einstellung angezeigt wird.

Im dargestellten Beispiel ist zu erkennen, dass die grafische Einstellhilfe dem Nutzer zumindest eine zweifache (siehe 31, 34), bevorzugt eine dreifache (siehe 31, 32, 34) Rückmeldung zur Einstellung gibt. Die Bedienvorrichtung für ein Beatmungsgerät umfasst eine berührungsempfindliche grafische Anzeige 3, 13, 14, 15, die zumindest zeitweise den Wertebereich für einen Beatmungsparameter 14a ... 14x, hier die Rampensteilheit 30, repräsentiert und zumindest einzelne Werte der Rampensteilheit numerisch darstellt (siehe 31). Darüber hinaus werden ein Speicher für den Wert der Rampensteilheit für mindestens einen mit dem Wertebereich verbundenen Datenpunkt und mindestens eine mit dem Datenpunkt schaltlogisch verbundene Stelle 14a ... 14x der berührungsempfindlichen grafischen Anzeige verwendet. Weiterhin sind vorgesehen: eine Schaltlogik 18, die bei Berührung der dem Datenpunkt schaltlogisch zugeordneten Stelle der berührungsempfindlichen grafischen Anzeige eine Anzeige von mindestens einem dem Datenpunkt zugeordneten Zahlenwert 32 und/oder eines Bestätigungsfeldes 33 für den Zahlenwert 32 veranlasst, und eine Schaltlogik 18, die diesen Zahlenwert 32 bei Berührung des Zahlenwertes 32 oder des Betätigungsfeldes 33 auf den zugeordneten Atemgasparameter anwendet und mit dem zugeordneten Atemgasparameter in den Speicher schreibt.

Das Bedienfeld ist in Form eines Zahlenstrahls oder Lineals ausgeführt. Der gesamte Wertebereich ist in Form eines Zahlenstrahls oder Balkens auf der Anzeige 13 visualisiert. Der visualisierte Zahlenstrahl ist auch als Bedienfeld (14f) ausgebildet. Das Bedienfeld ist über den gesamten visualisierten Einstellbereich berührungsempfindlich. Der gewünschte Wert ist mit lediglich einer Berührung des gewünschten Bereiches anwählbar.

Ein Fingerdruck oder eine Berührung innerhalb des Lineals wird bezüglich seiner Position derart ausgewertet, dass nicht genau eine der Zahlen "1", "2", "3" getroffen werden muss, sondern der Fingerdruck der nächstliegenden Zahl zugeordnet wird. Der erkannte Wert 32 wird in einem Zusatzfeld 14f1 visualisiert. Der erkannte Wert 32 kann zusätzlich mittels der Symbole +/- (siehe 14f2,14f3) verstellt werden.

Bevorzugt werden nicht nur einzelne Werte 31 der Rampensteilheit numerisch dargestellt, sondern es wird auch der gewählte Wert 33 der Rampensteilheit numerisch dargestellt. Auch erfolgt eine grafische Visualisierung 34 der gewählten Rampensteilheit.

Alternativ oder ergänzend kann, wie in Bezug auf Fig. 7a, 7b, 7c ausgeführt, auch die Rampe von dem inspiratorischen zu dem exspiratorischen Druck eingestellt werden.

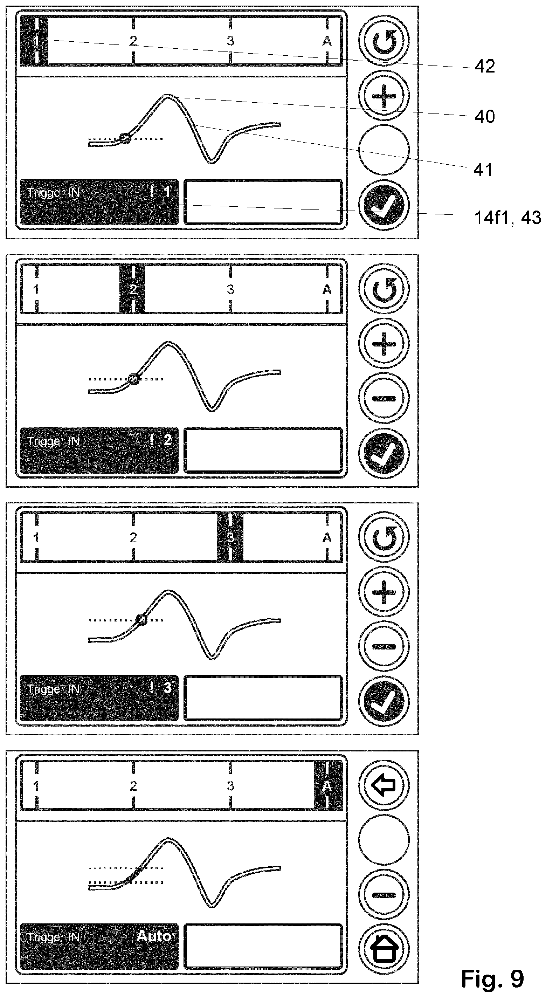

Fig. 9 zeigt analog zu Fig. 8 die Einstellung der Triggersensitivität 40. Der Schwellenwert 41 ist als grüne Linie schematisch dargestellt. Gegenüber Fig. 7a, 7b, 7c sieht man, dass im Slider oder Lineal auch Werte ausgewählt werden können, die keinen Zahlenwert darstellen, in diesem Fall "A" für die Stufe "Auto". Die Bedienvorrichtung für ein Beatmungsgerät umfasst eine berührungsempfindliche grafische Anzeige 3, 13, 14, 15, die zumindest zeitweise den Wertebereich für einen Beatmungsparameter 14a ... 14x, hier die Triggersensitivität 40, repräsentiert und zumindest einzelne Werte 42 der Triggersensitivität numerisch darstellt, einen Speicher für den Wert der Triggersensitivität und mindestens einen mit dem Wertebereich verbundenen Datenpunkt. Ebenfalls werden verwendet: mindestens eine mit dem Datenpunkt schaltlogisch verbundene Stelle 14a ... 14x der berührungsempfindlichen grafischen Anzeige und eine Schaltlogik 18, die bei Berührung der dem Datenpunkt schaltlogisch zugeordneten Stelle der berührungsempfindlichen grafischen Anzeige eine Anzeige von mindestens einem dem Datenpunkt zugeordneten Zahlenwert 32 und/oder eines Bestätigungsfeldes 33 für den Zahlenwert veranlasst. Ebenfalls vorgesehen ist eine Schaltlogik 18, die diesen Zahlenwert 32 bei Berührung des Zahlenwertes 32 oder des Betätigungsfeldes 33 auf den zugeordneten Atemgasparameter anwendet und mit dem zugeordneten Atemgasparameter in den Speicher schreibt.

Das Bedienfeld ist in Form eines Zahlenstrahls oder Lineals ausgeführt. Der gesamte Wertebereich ist in Form eines Zahlenstrahls oder Balkens auf der Anzeige 13 visualisiert und der visualisierte Zahlenstrahl ist auch als Bedienfeld 14f ausgebildet. Das Bedienfeld ist über den gesamten visualisierten Einstellbereich berührungsempfindlich und der gewünschte Wert ist mit lediglich einer Berührung des gewünschten Bereiches anwählbar. Ein Fingerdruck oder eine Berührung innerhalb des Lineals wird bezüglich seiner Position derart ausgewertet, dass nicht genau eine der Zahlen "1", "2", "3" getroffen werden muss, sondern der Fingerdruck der nächstliegenden Zahl zugeordnet wird. Der erkannte Wert 32 wird in einem Zusatzfeld 14f1 visualisiert.

Der erkannte Wert kann zusätzlich mittels der Symbole +/- verstellt werden. Bevorzugt werden nicht nur einzelne Werte 42 der Triggersensitivität numerisch dargestellt, sondern es wird auch der gewählte Wert 43 der Triggersensitivität numerisch dargestellt. Auch erfolgt eine grafische Visualisierung 41 der gewählten Triggersensitivität. Vorliegend sind drei feste Triggerstufen vorgesehen. Diese können jedoch auch mittels der Symbole +/- verstellt werden, um so den Trigger patientenindividuell feiner abzustimmen. Wird die Stufe "A" für die Stufe "Auto" gewählt, so verstellt sich der Trigger adaptiv innerhalb vorgegebener Grenzwerte, die auch grafisch visualisiert werden.

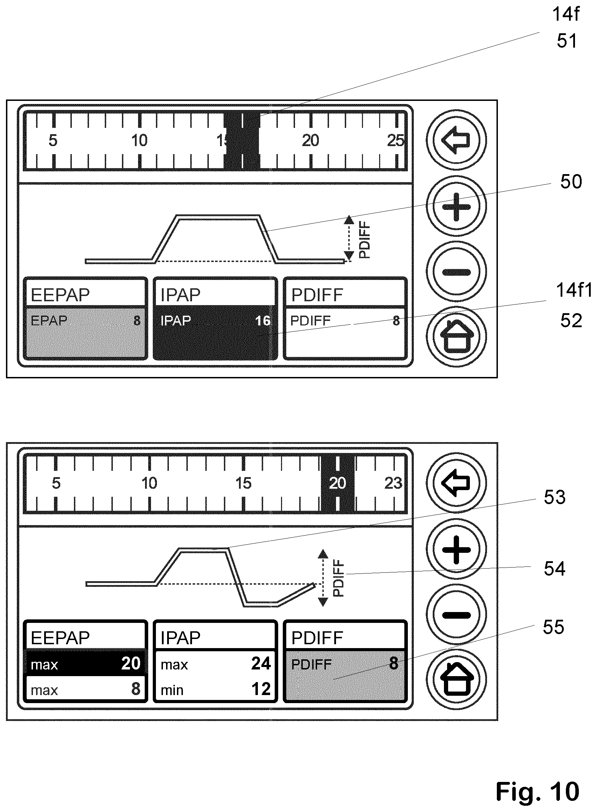

In Fig. 10 sieht man die grafische Einstellhilfe für mindestens einen inspiratorischen und einen exspiratorischen Druck. Durch die Position der Druckkachel unterhalb der Grafik werden der inspiratorische und der exspiratorische Druck sowie der Druckhub PDIFF (siehe auch 54) verdeutlicht. Die Farben der Kacheln stellen Folgendes dar:
- Grün: aktuell ausgewählter Parameter, der über Slider oder Plus/Minus-Tasten verstellt werden kann;
- Grau: Parameter, der alternativ zur Verstellung ausgewählt werden kann;
- Schwarz: informativ dargestellter Parameter, der sich als Konsequenz aus den Einstellungen ergibt.

Alternativ könnte im Slider nicht nur der Wert des aktuell einzustellenden Parameters in einer Farbe dargestellt werden, sondern zusätzlich die Werte weiterer Parameter mit einer Markierung, die sich in Form und/oder Farbe unterscheidet. Die Bedienvorrichtung für ein Beatmungsgerät umfasst eine berührungsempfindliche grafische Anzeige 3, 13, 14, 15, die zumindest zeitweise den Wertebereich für einen Beatmungsparameter 14a ... 14x, hier die Druckwerte 50 für IPAP und/oder EPAP und/oder EEPAP, repräsentiert und zumindest einzelne Werte 51 der Triggersensitivität numerisch darstellt, einen Speicher für den Druckwert, mindestens einen mit dem Wertebereich verbundenen Datenpunkt, mindestens eine mit dem Datenpunkt schaltlogisch verbundene Stelle 14a ... 14x der berührungsempfindlichen grafischen Anzeige, eine Schaltlogik 18, die bei Berührung der dem Datenpunkt schaltlogisch zugeordneten Stelle der berührungsempfindlichen grafischen Anzeige eine Anzeige von mindestens einem dem Datenpunkt zugeordneten Zahlenwert 52 und /oder eines Bestätigungsfeldes für den Zahlenwert veranlasst, eine Schaltlogik 18, die diesen Zahlenwert 52 bei Berührung des Zahlenwertes 52 oder des Betätigungsfeldes auf den zugeordneten Atemgasparameter anwendet und mit dem zugeordneten Atemgasparameter in den Speicher schreibt.

Das Bedienfeld ist in Form eines Zahlenstrahls oder Lineals ausgeführt. Der gesamte Wertebereich ist in Form eines Zahlenstrahls oder Balkens auf der Anzeige 13 visualisiert und der visualisierte Zahlenstrahl ist auch als Bedienfeld 14f ausgebildet. Das Bedienfeld ist über den gesamten visualisierten Einstellbereich berührungsempfindlich und der gewünschte Wert ist mit lediglich einer Berührung des gewünschten Bereiches anwählbar. Ein Fingerdruck oder eine Berührung innerhalb des Lineals wird bezüglich seiner Position derart ausgewertet, dass nicht genau eine der Zahlen getroffen werden muss, sondern der Fingerdruck der nächstliegenden Zahl zugeordnet wird. Der erkannte Wert 52 wird in einem Zusatzfeld 14f1 visualisiert.

Der erkannte Wert kann zusätzlich mittels der Symbole +/- verstellt werden. Bevorzugt werden nicht nur einzelne Druckwerte 52 numerisch dargestellt, sondern es wird auch der gewählte Wert numerisch dargestellt. Auch erfolgt eine grafische Visualisierung 53 des gewählten Druckes. Dieser kann jedoch auch mittels der Symbole +/verstellt werden. Bevorzugt wird zur Information auch der resultierende Druckhub als numerischer Wert 55 dargestellt und grafisch visualisiert (siehe 54).

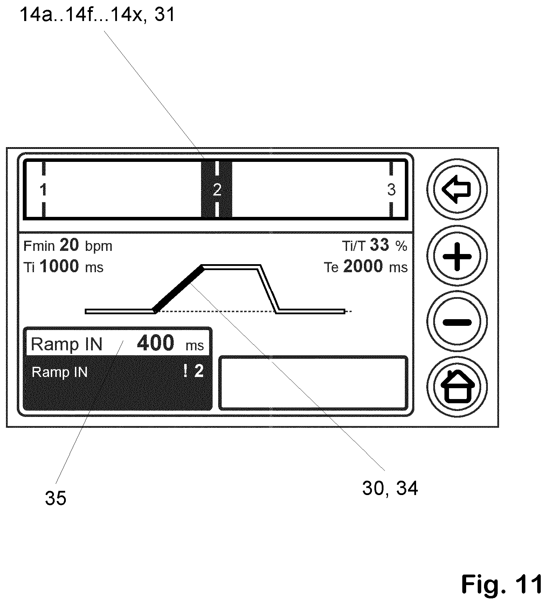

In Fig. 11 wird als Ergänzung zu Fig. 8 in Kombination mit der aktuellen Rampensteilheit "1", "2" oder "3" (siehe 31 in Fig. 8) noch die Einstellung weiterer, damit in logischem Zusammenhang stehender Parameter informativ dargestellt. Dadurch kann der Anwender den aktuell einzustellenden Wert sinnvoll wählen, ohne sich die übrigen Parameter merken zu müssen. Als Konsequenz aus der aktuellen Rampensteilheit und den übrigen Parametern wird die aktuell gültige Rampenzeit in ms (siehe 35 in Fig. 11) berechnet und ebenfalls informativ dargestellt.

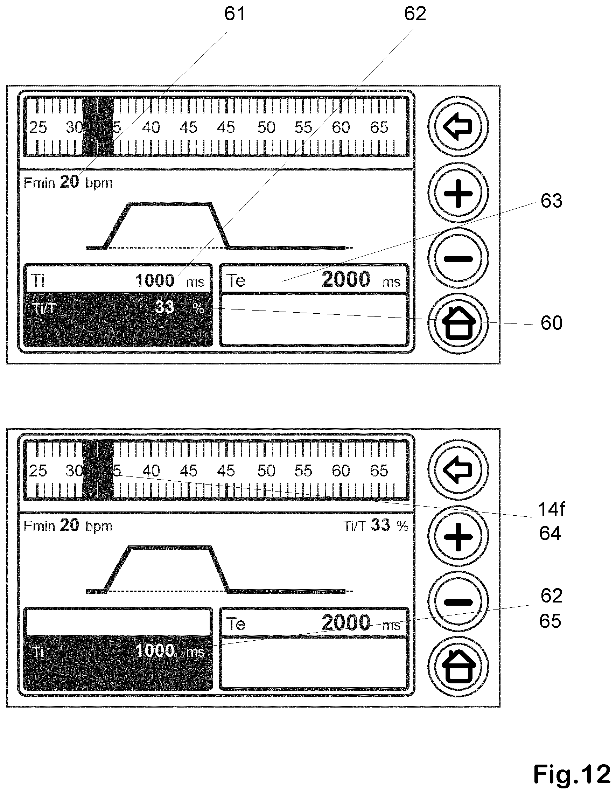

Fig. 12 zeigt eine Einstellung eines Verhältnisses "Ti/T" der inspiratorischen Zeit zur gesamten Atemzugszeit in % (siehe 60). Zusätzlich ist eine gewählte Atemfrequenz "Fmin" (siehe 61) dargestellt. Als Konsequenz aus der eingestellten Atemfrequenz "Fmin" und dem eingestellten Verhältnis "Ti/T" werden die Inspirationsdauer "Ti" (siehe 62) und die Exspirationsdauer "Te" (siehe 63) berechnet und informativ dargestellt.

In der unteren Hälfte von Fig. 12 ist eine alternative Ausführung dargestellt, bei der die Inspirationsdauer "Ti" eingestellt wird. In Kombination mit der gewählten Atemfrequenz "Fmin" ergeben sich daraus die Exspirationsdauer "Te" und das Verhältnis "Ti/T" automatisch. Die Bedienvorrichtung für ein Beatmungsgerät umfasst eine berührungsempfindliche grafische Anzeige 3, 13, 14, 15, die zumindest zeitweise den Wertebereich für einen Beatmungsparameter 14a ... 14x, hier die Inspirationsdauer 62, repräsentiert und zumindest einzelne Werte 64 numerisch darstellt, einen Speicher für die Inspirationsdauer, mindestens einen mit dem Wertebereich verbundenen Datenpunkt, mindestens eine mit dem Datenpunkt schaltlogisch verbundene Stelle 14a ... 14x der berührungsempfindlichen grafischen Anzeige, eine Schaltlogik 18, die bei Berührung der dem Datenpunkt schaltlogisch zugeordneten Stelle der berührungsempfindlichen grafischen Anzeige eine Anzeige von mindestens einem dem Datenpunkt zugeordneten Zahlenwert 65 und/oder eines Bestätigungsfeldes für den Zahlenwert veranlasst, eine Schaltlogik 18, die diesen Zahlenwert 65 bei Berührung des Zahlenwertes 65 oder des Betätigungsfeldes auf den zugeordneten Atemgasparameter anwendet und mit dem zugeordneten Atemgasparameter in den Speicher schreibt.

Das Bedienfeld ist in Form eines Zahlenstrahls oder Lineals ausgeführt. Der gesamte Wertebereich ist in Form eines Zahlenstrahls oder Balkens auf der Anzeige 13 visualisiert und der visualisierte Zahlenstrahl ist auch als Bedienfeld 14f ausgebildet. Das Bedienfeld ist über den gesamten visualisierten Einstellbereich berührungsempfindlich und der gewünschte Wert ist mit lediglich einer Berührung des gewünschten Bereiches anwählbar.

Ein Fingerdruck oder eine Berührung innerhalb des Lineals wird bezüglich seiner Position derart ausgewertet, dass nicht genau eine der Zahlen getroffen werden muss, sondern der Fingerdruck der nächstliegenden Zahl zugeordnet wird. Der erkannte Wert wird in einem Zusatzfeld visualisiert. Der erkannte Wert kann zusätzlich mittels der Symbole +/- verstellt werden. Bevorzugt werden nicht nur einzelne Werte numerisch dargestellt, sondern es wird auch der gewählte Wert numerisch dargestellt. Auch erfolgt eine grafische Visualisierung des gewählten Wertes. Dieser kann jedoch auch mittels der Symbole +/- verstellt werden. Bevorzugt wird zur Information auch die resultierende Ausatemzeit 63 als numerischer Wert dargestellt und/oder grafisch visualisiert.

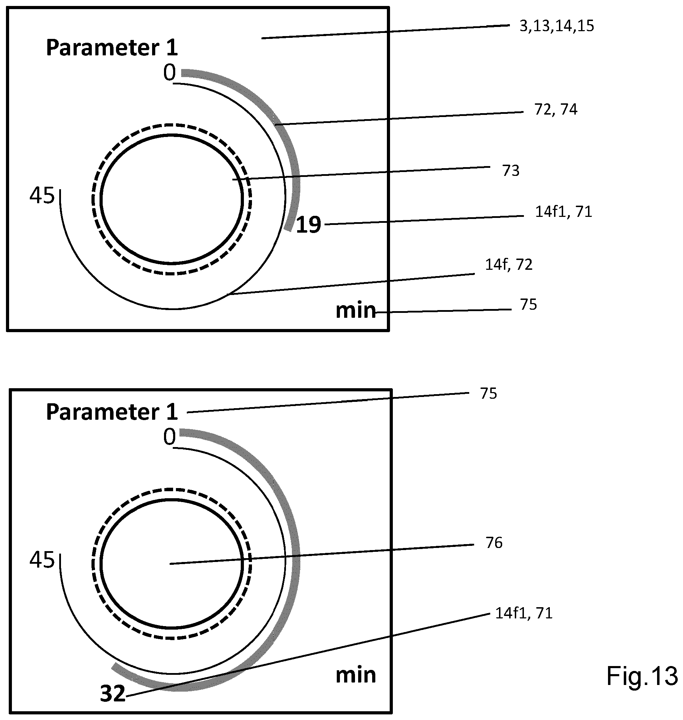

Fig. 13 zeigt die beschriebene kreisförmige Ausführung des Bedienelementes. Auf der berührungsempfindlichen Anzeige 13 wird ein Drehrad 73 simuliert, welches mit dem Finger bedient werden kann. Um das Bedienfeld herum ist ein ebenfalls kreisförmiges Anzeigeelement 72 platziert, welches mindestens den aktuell eingestellten Wert 71 darstellt, im Beispiel 19 Minuten, besonders bevorzugt auch die Wertegrenzen bzw. den Wertebereich, im Beispiel 0 bis 45 Minuten. Die Darstellung erfolgt als Zahl und, besonders bevorzugt, zusätzlich durch eine farbige und/oder dickere Markierung 74, die die aktuellen Werte in Relation zum gesamten Wertebereich darstellt.

Die Bedienvorrichtung für ein Beatmungsgerät umfasst eine berührungsempfindliche grafische Anzeige 3, 13, 14, 15, die zumindest zeitweise den Wertebereich für einen Beatmungsparameter 14a ... 14x repräsentiert und zumindest einzelne Werte 71 numerisch darstellt, einen Speicher für mindestens einen mit dem Wertebereich verbundenen Datenpunkt, mindestens eine mit dem Datenpunkt schaltlogisch verbundene Stelle 14a ... 14x der berührungsempfindlichen grafischen Anzeige, eine Schaltlogik 18, die bei Berührung der dem Datenpunkt schaltlogisch zugeordneten Stelle der berührungsempfindlichen grafischen Anzeige eine Anzeige von mindestens einem dem Datenpunkt zugeordneten Zahlenwert 71 und/oder eines Bestätigungsfeldes für den Zahlenwert veranlasst, eine Schaltlogik 18, die diesen Zahlenwert 71 bei Berührung des Zahlenwertes 71 oder des Betätigungsfeldes auf den zugeordneten Atemgasparameter anwendet und mit dem zugeordneten Atemgasparameter in den Speicher schreibt.

Das Bedienfeld ist in Form eines Drehknopfes 73 oder Drehrades 73 ausgeführt. Der gesamte Wertebereich oder ein Teil davon ist in Form eines Zahlenkranzes 72 auf der Anzeige 13 visualisiert. Der visualisierte Zahlenkranz 72 ist auch als Bedienfeld 14f ausgebildet. Das Bedienfeld 14f ist über den gesamten visualisierten Einstellbereich berührungsempfindlich. Der gewünschte Wert ist mit lediglich einer Berührung des gewünschten Bereiches anwählbar. Ein Fingerdruck oder eine Berührung innerhalb des Zahlenkranzes 72 wird bezüglich seiner Position derart ausgewertet, dass nicht genau eine der Zahlen getroffen werden muss, sondern der Fingerdruck der nächstliegenden Zahl zugeordnet wird.

Alternativ wird ein Streichen über den Zahlenkranz 72 als Einstellvorgang und ein Stoppen der Streichbewegung als Auswahl erkannt. Derjenige Wert, der beim Stoppen der Bewegung erkannt wird, der erkannte Wert 71, wird in einem Zusatzfeld 14f1 visualisiert. Der erkannte Wert kann zusätzlich mittels der Symbole +/- verstellt werden. Bevorzugt werden nicht nur einzelne Werte numerisch dargestellt, sondern es wird auch der gewählte Wert numerisch dargestellt. Auch erfolgt eine grafische Visualisierung des gewählten Wertes. Dieser kann jedoch auch mittels der Symbole +/verstellt werden.

Besonders bevorzugt wird der aktuell einzustellende Parameter 75 mit einem Namen und/oder einem international verständlichen Symbol und/oder seiner Einheit dargestellt. Im dargestellten Beispiel könnte der Parameter die Rampenzeit als Einschlafhilfe eines Therapiegerätes sein, die in Minuten dargestellt wird. Alternativ dazu können u. a. Therapiedrücke oder Leistungsstufen eines Atemluftbefeuchters dargestellt und verstellt werden.

Führt der Anwender mit dem Finger eine Drehbewegung auf dem dargestellten Drehrad 73 aus, vorzugsweise im Uhrzeigersinn, so vergrößert sich der gewählte Wert, im Beispiel von 19 auf 32 Minuten. Eine Drehbewegung in die Gegenrichtung führt zu einer Verringerung des gewählten Wertes. Ist der beabsichtigte Wert erreicht, kann er übernommen und vom Gerät angewendet werden. Dies erfolgt typischerweise entweder nach Ablauf einer Wartezeit ohne weitere Verstellung oder nach Drücken einer Bestätigungstaste oder einer Bestätigungsfläche 76, die z. B. mit "Übernehmen", "Ok", "Anwenden", einem Hakensymbol oder Ähnlichem gekennzeichnet ist. Diese befindet sich besonders bevorzugt in der Mitte des dargestellten Drehrades 73.

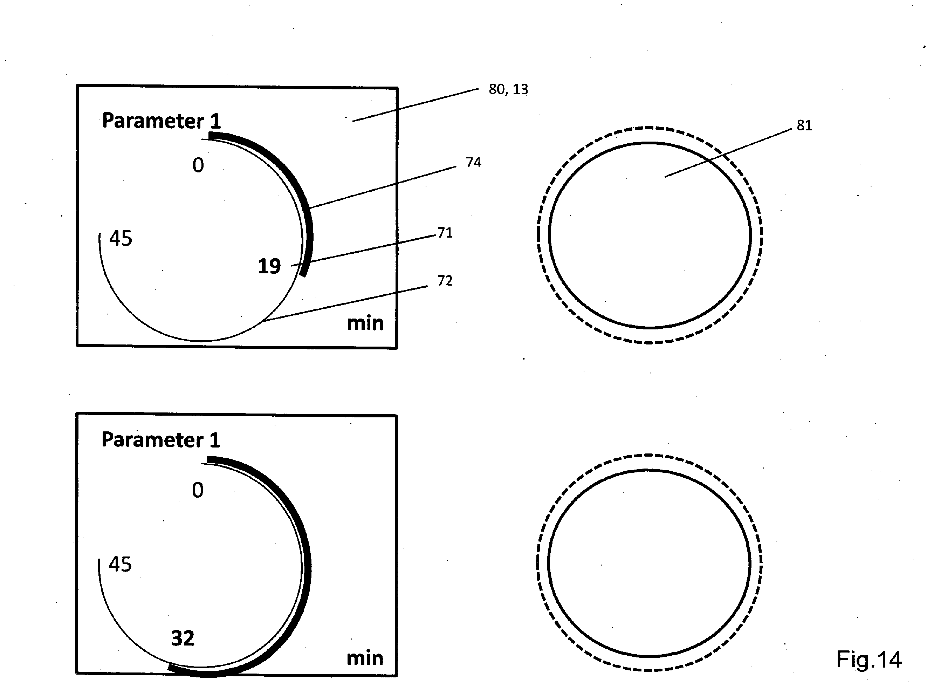

Fig. 14 zeigt eine alternative Ausführungsform. Hier befinden sich das Bedienelement "Drehrad" und das kreisförmige Anzeigeelement nebeneinander oder untereinander. Dies ist eine bevorzugte Ausführungsform, falls kein berührungsempfindliches Anzeigeelement verwendet wird, sondern nur ein grafisches Anzeigeelement 80 mit einem separat ausgeführten mechanischen Dreh-Drück-Knopf 81. Diese Ausführungsform bietet einen Vorteil, dass der mechanische Dreh-Drück-Knopf 81 wegen der Haptik oder besseren Bedienbarkeit bevorzugt für feine Einstellungen verwendet werden kann. Die vom mechanischen Dreh-Drück-Knopf 81 entkoppelte grafische Visualisierung des Einstellvorganges und/oder der ausgewählten Wertes und/oder des zur Verfügung stehenden Wertebereiches bietet den Vorteil, dass eine größere und verbesserte Darstellung gewählt werden kann, als es eine Skala neben dem mechanischen Dreh-Drück-Knopf 81 bieten könnte.

Das Bestätigen eines gewählten Wertes geschieht hier besonders bevorzugt durch Drücken des Dreh-Drück-Knopfes 81.

Ansonsten ist die Art der Darstellung und Einstellung mit dem Ausführungsbeispiel von Fig. 13 vergleichbar, weshalb die Beschreibung zu Fig. 13 auch für das Beispiel von Fig. 14 herangezogen werden kann.

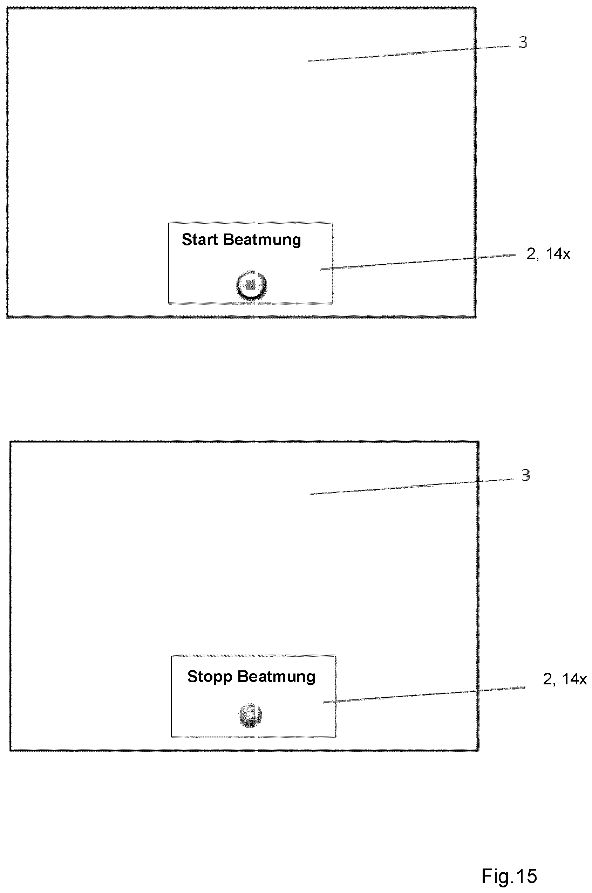

Erfindungsgemäß kann eine (Start/Stopp-)Bedienfläche 14x auf dem Touchscreen oder ein mechanisches (Start/Stopp-)Bedienelement 2 gemäß Fig. 15 vorgesehen sein, dessen Betätigung das Bedien- und Informationssystem 3 veranlasst, über die Steuereinheit die Beatmung zu starten oder zu beenden.

Erfindungsgemäß ist vorgesehen, dass die (Start/Stopp-)Bedienfläche 14x auf dem Touchscreen situationsabhängig unterschiedlich gestaltet ist. Wenn beispielsweise die Beatmung nicht aktiv ist, aber gestartet werden kann, ist die (Start/Stopp-)Bedienfläche 14x auf dem Touchscreen beispielsweise zumindest teilweise grün eingefärbt oder weist ein Startsymbol und beispielsweise ergänzend eine schriftliche Information "Beatmung Start" auf. Wenn die Beatmung aktiv ist und gestoppt werden kann, ist die (Start/Stopp-)Bedienfläche 14x auf dem Touchscreen zumindest teilweise rot eingefärbt oder weist ein Stoppsymbol und beispielsweise ergänzend eine schriftliche Information "Beatmung Stopp" auf.

Dabei ist beispielsweise vorgesehen, dass die (Start/Stopp-)Bedienfläche 14x ansonsten unverändert an immer derselben Position auf dem Touchscreen und/oder in immer derselben Größe erscheint.

Bei Bestätigung der (Start/Stopp-)Bedienfläche 14x auf dem Touchscreen zum Stoppen der Beatmung ("Beatmung Stopp") werden von der Steuereinheit aktuelle Einstellungen für die Beatmung, wie beispielsweise aktuelle Druckwerte, abrufbar gespeichert und bei Wiederbetätigung der (Start/Stopp-)Bedienfläche 14x zum Starten der Beatmung wieder ausgelesen und für die Beatmung aktiviert, insbesondere sofern während des Beatmungsstopps keine Veränderungen an den Einstellungen für die Beatmung vorgenommen wurden.

## Patentansprüche

1. Bedienvorrichtung für ein Beatmungsgerät, wobei die Bedienvorrichtung umfasst:
eine berührungsempfindliche grafische Anzeige (3, 13, 14, 15), die ausgebildet ist, um zumindest zeitweise einen Wertebereich für einen Beatmungsparameter (14a ... 14x) zu repräsentieren und zumindest einzelne Werte des Wertebereichs numerisch darzustellen;
einen Speicher für Werte des Beatmungsparameters;
mindestens einen mit dem Wertebereich verbundenen Datenpunkt, wobei die Anzeige an mindestens einer Stelle (14a ... 14x) mit dem Datenpunkt schaltlogisch verbunden ist;
eine Schaltlogik (18), die ausgebildet ist, um bei Berührung der dem Datenpunkt schaltlogisch zugeordneten Stelle der Anzeige zu veranlassen, dass mindestens ein dem Datenpunkt zugeordneter Zahlenwert und/oder ein Bestätigungsfeld für einen dem Datenpunkt zugeordneten Zahlenwert angezeigt wird, und den Zahlenwert bei Berührung des Zahlenwerts oder des Bestätigungsfeldes auf den zugeordneten Beatmungsparameter anzuwenden und zusammen mit dem zugeordneten Beatmungsparameter in den Speicher zu schreiben;
wobei auf der Anzeige (13) ein Bedienfeld (14f) in Form eines den gesamten Wertebereich visualisierenden Zahlenstrahls zum Einstellen von Zahlenwerten und eines weiteren optischen Feedbackbereichs separat zum Zahlenstrahl ausgeführt ist, wobei das Bedienfeld (14f) über dem gesamten visualisierten Wertebereich berührungsempfindlich ist und ein gewünschter Wert des Wertebereichs mit lediglich einer Berührung eines Bereichs des Bedienfelds (14f) anwählbar ist;
wobei die Bedienvorrichtung so ausgebildet ist, dass eine Rampensteigung für eine Inspiration-Exspiration-Rampe mit dem Zahlenstrahl eingestellt werden kann und - wenn die Rampensteigung für die Inspiration-Exspiration-Rampe eingestellt wird - auf der Anzeige an einer weiteren Position ein Trapez als Vereinfachung eines Druckprofils dargestellt wird, in dem die Inspiration-Exspiration-Rampe analog zu einer Auswahl auf dem Bedienfeld steiler oder flacher dargestellt wird;
wobei die Bedienvorrichtung ferner ausgebildet ist, um nicht nur einzelne Werte (31) der Rampensteigung, sondern auch einen gewählten Wert der Rampensteigung numerisch darzustellen und den gewählten Wert (34) grafisch zu visualisieren;
wobei die Bedienvorrichtung ferner ausgebildet ist, um in Kombination mit einer aktuellen Rampensteigung eine Einstellung damit in logischem Zusammenhang stehender weiterer Parameter informativ darzustellen und als Konsequenz aus der aktuellen Rampensteigung und den übrigen Parametern eine aktuell gültige Rampenzeit (35) in ms zu berechnen und ebenfalls informativ darzustellen.

2. Bedienvorrichtung nach Anspruch 1,
wobei die Bedienvorrichtung so ausgebildet ist, dass ein erkannter Wert des Wertebereichs in einem Zusatzfeld (14f1) visualisiert wird und der erkannte Wert mittels eines Plussymbols (14f2) und eines Minussymbols (14f3) einer Feineinstellung zugänglich ist.

3. Bedienvorrichtung nach einem der vorhergehenden Ansprüche,
wobei die Bedienvorrichtung so ausgebildet ist, dass eine Charakteristik einer Druckanhebung oder -absenkung rampenförmig einstellbar ist.

4. Bedienvorrichtung nach Anspruch 3,
wobei die Druckanhebung durch Anhebung des Drucks mit einer gleichmäßigen Rampensteigung auf ein erhöhtes Druckniveau erfolgt.

5. Bedienvorrichtung nach Anspruch 3 oder 4,
wobei die Druckanhebung oder -absenkung mit einer veränderlichen Rampensteigung erfolgt.

6. Bedienvorrichtung nach einem der vorhergehenden Ansprüche,
wobei die Bedienvorrichtung so ausgebildet ist, dass eine Geschwindigkeit eines Druck- oder Flussanstiegs und somit eine Dauer des Druckanstiegs von einem unteren auf ein oberes Druckniveau festgelegt werden kann.

7. Bedienvorrichtung nach einem der vorhergehenden Ansprüche,
wobei die Bedienvorrichtung ausgebildet ist, um einen Fluss einer Atemluft einzustellen, sodass zu Beginn einer Inspiration die Atemluft mit einem geringeren Fluss als dem eingestellten Fluss verabreicht wird und der Fluss im Verlauf der Inspiration bis auf den eingestellten Wert zunimmt.

8. Bedienvorrichtung nach einem der vorhergehenden Ansprüche,
wobei eine grafische Einstellhilfe für eine Rampensteigung für einen Übergang von einem exspiratorischen Druck auf einen inspiratorischen Druck vorgesehen ist, wobei die grafische Einstellhilfe so ausgebildet ist, dass eine aktuelle Stufe der Rampensteigung per Slider, Lineal oder Plus- und Minussymbol eingestellt werden kann.

9. Bedienvorrichtung nach Anspruch 8,
wobei die grafische Einstellhilfe ausgebildet ist, um einem Nutzer zumindest eine zweifache Rückmeldung zur Einstellung zu geben.

10. Bedienvorrichtung nach einem der vorhergehenden Ansprüche,
wobei ein Bedienfeld in Form einer Grafik vorgesehen ist, wobei die Grafik aktuelle oder gespeicherte Druckwerte für einen inspiratorischen Druck, einen exspiratorischen Druck und ein endexspiratorisches Druckniveau visualisiert und zudem Steigungen von Druckübergängen und eine Druckwellenform darstellt.

11. Bedienvorrichtung nach einem der vorhergehenden Ansprüche,
wobei die Bedienvorrichtung so ausgebildet ist, dass ein Anwender eine Steigung eines Druckübergangs von einem exspiratorischen positiven Druck auf einen inspiratorischen positiven Druck verändern kann, indem er eine Linie (14k), die die Steigung repräsentiert, berührt und dann durch Verschieben mit einem Finger in horizontaler Richtung auf eine gewünschte Steigung bringt.

12. Bedienvorrichtung nach Anspruch 11,
wobei die gewählte Linie (14k) als unterbrochene Linie mit der Bewegung mitwandert, wobei ein gegenüberliegender Ankerpunkt der Linie fixiert bleibt, wobei - wenn der Anwender die Berührung beendet - der eingestellte Wert angewendet wird und die Linie wieder durchgezogen dargestellt wird.

13. Bedienvorrichtung nach einem der vorhergehenden Ansprüche,
wobei die Bedienvorrichtung so ausgebildet ist, dass ein Anwender eine Druckwellenform (14m) einstellen kann, wobei die Druckwellenform (14m) eine Überhöhung eines inspiratorischen positiven Drucks (14g) ist.

## Claims

1. An operating device for a ventilator, wherein the operating device comprises:
a touch-sensitive graphic display (3, 13, 14, 15) which is designed to at least temporarily represent a value range for a ventilation parameter (14a ... 14x) and numerically display at least some values in the value range;
a storage unit for values of the ventilation parameter;
at least one data point connected with the value range, wherein the display is connected via control logic to the data point at at least one location (14a ... 14x);
control logic (18) which is designed to cause at least one number value associated with the data point and/or a confirmation field for a number value associated with the data point to be displayed when the location on the display associated by control logic with the data point is touched, and to apply the number value to the associated ventilation parameter when the number value or the confirmation field is touched and to write the number value to the storage unit together with the associated ventilation parameter;
wherein an operating field (14f) is implemented on the display (13) in the form of a number line visualizing the entire value range for setting number values and an additional optical feedback region which is separate from the number line, wherein the operating field (14f) is touch-sensitive over the entire visualized value range and a desired value in the value range can be selected just by touching a region of the operating field (14f);
wherein the operating device is designed such that a ramp slope for an inspiration/expiration ramp can be set using the number line and - when the ramp slope is set for the inspiration/expiration ramp - a trapezoid is displayed on the display at another position as a simplification of a pressure profile by displaying the inspiration/expiration ramp steeper or flatter analogously to a selection on the operating field;
wherein the operating device is also designed not only to display individual values (31) of the ramp slope but also to numerically display a selected value of the ramp slope and graphically visualize the selected value (34);
wherein the operating device is also designed to display, for informational purposes and in combination with a current ramp slope, a setting of further parameters logically related thereto and consequently to calculate a currently valid ramp time (35) in ms from the current ramp slope and the remaining parameters and also to display it for informational purposes.

2. The operating device according to claim 1,
wherein the operating device is designed such that a detected value in the value range is visualized in an additional field (14f1) and the detected value can be finely adjusted by means of a plus symbol (14f2) and a minus symbol (14f3).

3. The operating device according to one of the preceding claims,
wherein the operating device is designed such that a characteristic of a pressure increase or decrease can be set in the form of a ramp.

4. The operating device according to claim 3,
wherein the pressure increase to an increased pressure level takes place by increasing the pressure with a uniform ramp slope.

5. The operating device according to claim 3 or 4,
wherein the pressure increase or decrease takes place with a variable ramp slope.

6. The operating device according to one of the preceding claims,
wherein the operating device is designed such that a speed of a rise in pressure or flow and thus a duration of the rise in pressure from a lower to an upper pressure level can be specified.

7. The operating device according to one of the preceding claims,
wherein the operating device is designed to set a flow of respiratory air such that, at the beginning of an inspiration, the respiratory air is administered with a lower flow than the set flow, and the flow increases up to the set value over the course of the inspiration.

8. The operating device according to one of the preceding claims,
wherein a graphical setting aid for a ramp slope for a transition from an expiratory pressure to an inspiratory pressure is provided, wherein the graphical setting aid is designed such that a current stage of the ramp slope can be set by means of a slider, ruler, or plus and minus symbols.

9. The operating device according to claim 8,
wherein the graphical setting aid is designed to give a user at least a twofold feedback regarding the setting.

10. The operating device according to one of the preceding claims,
wherein an operating field is provided in the form of a graphic, wherein the graphic visualizes current or stored pressure values for an inspiratory pressure, an expiratory pressure, and an end expiratory pressure level and additionally displays slopes of pressure transitions and a pressure waveform.

11. The operating device according to one of the preceding claims,
wherein the operating device is designed such that an operator can modify a slope of a pressure transition from a positive expiratory pressure to a positive inspiratory pressure by touching a line (14k) representing the slope and then bringing it to a desired slope by shifting it in the horizontal direction with a finger.

12. The operating device according to claim 11,
wherein the selected line (14k) moves along with the movement as a broken line, wherein an opposite anchor point of the line remains fixed, wherein - when the operator stops touching the line - the set value is applied and the line is displayed again as a solid line.

13. The operating device according to one of the preceding claims,
wherein the operating device is designed such that an operator can set a pressure waveform (14m), wherein the pressure waveform (14m) is an excess increase of a positive inspiratory pressure (14g).

## Revendications

1. Dispositif de commande pour un appareil respiratoire, le dispositif de commande comportant :
un moyen d'affichage graphique tactile (3, 13, 14, 15) conçu pour représenter au moins temporairement une plage de valeurs pour un paramètre respiratoire (14a ... 14x) et pour afficher numériquement au moins des valeurs individuelles de la plage de valeurs ;
une mémoire pour des valeurs du paramètre respiratoire ;
au moins un point de données lié à la plage de valeurs, dans lequel le moyen d'affichage est lié en au moins une zone (14a ... 14x) au point de données par une logique de commutation ;
une logique de commutation (18) réalisée pour entraîner, lors du toucher de la zone du moyen d'affichage associée au point de données par la logique de commutation, l'affichage d'au moins une valeur numérique associée au point de données et/ou d'un champ d'actionnement d'une valeur numérique associée au point de données, et pour appliquer la valeur numérique au paramètre respiratoire associé et l'écrire dans la mémoire conjointement avec le paramètre respiratoire associé lors du toucher de la valeur numérique ou du champ d'actionnement ;
dans lequel un champ de commande (14f) est réalisé sur le moyen d'affichage (13) sous la forme d'une droite numérique visualisant l'ensemble de la plage de valeurs pour le réglage de valeurs numériques et d'une autre zone de rétroaction optique séparée de la droite numérique, dans lequel le champ de commande (14f) est tactile sur l'ensemble de la plage de valeurs visualisée et une valeur souhaitée de la plage de valeurs peut être sélectionnée en touchant simplement une zone du champ de commande (14f) ;
dans lequel le dispositif de commande est réalisé de telle façon qu'une pente de rampe d'une rampe d'inspiration-expiration peut être réglée à l'aide de la droite numérique et - lors du réglage de la pente de rampe d'une rampe d'inspirationexpiration - un trapèze est affiché à une autre position sur le moyen d'affichage en tant que simplification d'un profil de pression, dans lequel la rampe d'inspiration-expiration est représentée plus raide ou plus plate en fonction d'une sélection sur le champ de commande ;
dans lequel le dispositif de commande est en outre réalisé pour afficher non seulement des valeurs (31) individuelles de la pente de rampe, mais aussi de façon numérique une valeur sélectionnée de la pente de rampe et pour visualiser graphiquement la valeur sélectionnée (34) ;
dans lequel le dispositif de commande est en outre réalisé pour afficher de façon informative, en combinaison avec une pente de rampe actuelle, un réglage d'autres paramètres en rapport logique avec celle-ci et pour calculer en ms et également afficher de façon informative un temps de rampe (35) actuellement valable en conséquence de la pente de rampe actuelle et des autres paramètres.

2. Dispositif de commande selon la revendication 1,
dans lequel le dispositif de commande est réalisé de telle façon qu'une valeur détectée de la plage de valeurs est visualisée dans un champ supplémentaire (14f1) et la valeur détectée est accessible au moyen d'un symbole plus (14f2) et d'un symbole moins (14f3) d'un réglage fin.

3. Dispositif de commande selon l'une des revendications précédentes,
dans lequel le dispositif de commande est réalisé de telle façon qu'une caractéristique d'une augmentation ou baisse de pression peut être réglée sous forme de rampe.

4. Dispositif de commande selon la revendication 3,
dans lequel l'augmentation de pression est effectuée par augmentation de la pression à un niveau de pression accru avec une pente de rampe régulière.

5. Dispositif de commande selon la revendication 3 ou 4,
dans lequel l'augmentation ou la baisse de pression est effectuée avec une pente de rampe modifiable.

6. Dispositif de commande selon l'une des revendications précédentes,
dans lequel le dispositif de commande est réalisé de manière à pouvoir définir une vitesse de la hausse de pression ou de flux et donc une durée de la hausse de pression d'un niveau de pression inférieur à un niveau de pression supérieur.

7. Dispositif de commande selon l'une des revendications précédentes,
dans lequel le dispositif de commande est réalisé pour régler un flux de l'air de respiration, de telle façon qu'au début d'une inspiration, l'air de respiration est délivré avec un flux plus faible que le flux réglé et le flux augmente au cours de l'inspiration jusqu'à la valeur réglée.

8. Dispositif de commande selon l'une des revendications précédentes,
dans lequel il est prévu une aide au réglage graphique d'une pente de rampe pour une transition à partir d'une pression expiratoire vers une pression inspiratoire, dans lequel l'aide au réglage graphique est réalisée de telle façon qu'un niveau actuel de la pente de rampe peut être réglé à l'aide d'un curseur, d'une règle ou d'un symbole plus et moins.

9. Dispositif de commande selon la revendication 8,
dans lequel l'aide au réglage graphique est réalisée pour fournir au moins un double retour concernant le réglage à un utilisateur.

10. Dispositif de commande selon l'une des revendications précédentes,
dans lequel il est prévu un champ de commande sous la forme d'un graphique, dans lequel le graphique visualise des valeurs de pression actuelles ou enregistrées pour une pression inspiratoire, une pression expiratoire et un niveau de pression de fin d'expiration, et affiche en outre des pentes de transitions de pression et une forme d'onde de pression.

11. Dispositif de commande selon l'une des revendications précédentes,
dans lequel le dispositif de commande est réalisé de telle façon qu'un utilisateur peut modifier une pente d'une transition de pression à partir d'une pression positive expiratoire vers une pression positive inspiratoire en touchant une ligne (14k) représentant la pente et en l'amenant ensuite à une pente souhaitée par décalage avec un doigt dans une direction horizontale.

12. Dispositif de commande selon la revendication 11,
dans lequel la ligne (14k) sélectionnée avance sous forme de ligne en pointillés conjointement avec le déplacement, dans lequel un point d'ancrage opposé de la ligne demeure fixe, dans lequel - lorsque l'utilisateur termine le toucher - la valeur réglée est appliquée et la ligne s'affiche de nouveau en continu.

13. Dispositif de commande selon l'une des revendications précédentes,
dans lequel le dispositif de commande est réalisé de telle façon qu'un utilisateur peut régler une forme d'onde de pression (14m), dans lequel la forme d'onde de pression (14m) est une surélévation d'une pression positive inspiratoire (14g).
